(19) Europäisches Patentamt
European Patent Office
Office européen des brevets

(11) EP 1 751 057 B1

# (12) EUROPEAN PATENT SPECIFICATION

(45) Date of publication and mention
of the grant of the patent:
**19.01.2011 Bulletin 2011/03**

(21) Application number: **04750373.5**

(22) Date of filing: **20.04.2004**

(51) Int Cl.:
*C01B 39/46* (2006.01)   *C01B 39/48* (2006.01)
*C01B 39/02* (2006.01)   *C01B 39/06* (2006.01)
*B01J 29/70* (2006.01)   *B01J 29/88* (2006.01)
*B01J 29/89* (2006.01)

(86) International application number:
**PCT/US2004/012152**

(87) International publication number:
**WO 2005/113439 (01.12.2005 Gazette 2005/48)**

(54) **UZM-8 AND UZM-8HS CRYSTALLINE ALUMINOSILICATE ZEOLITIC COMPOSITIONS AND PROCESSES USING THE COMPOSITIONS**

UZM-8- UND UZM-8HS-ZEOLITHZUSAMMENSETZUNGEN AUF BASIS VON KRISTALLINEM ALUMINOSILICAT UND HERSTELLUNGSVERFAHREN DAFÜR

COMPOSITIONS ZEOLITIQUES D'ALUMINOSILICATES CRISTALLINS UZM-8 ET UZM-8HS ET PROCESSUS UTILISANT CES COMPOSITIONS

(84) Designated Contracting States:
**AT BE BG CH CY CZ DE DK EE ES FI FR GB GR HU IE IT LI LU MC NL PL PT RO SE SI SK TR**

(43) Date of publication of application:
**14.02.2007 Bulletin 2007/07**

(73) Proprietor: **UOP LLC**
**Des Plaines, IL 60016-6100 (US)**

(72) Inventors:
• **KNIGHT, Lisa, M.**
**UOP LLC**
**Des Plaines, IL 60016 (US)**
• **LEWIS, Gregory, J.**
**UOP LLC**
**Des Plaines, IL 60016 (US)**
• **MILLER, Mark, A.**
**UOP LLC**
**Des Plaines, IL 60016 (US)**
• **MOSCOSO, Jamie, G.**
**UOP LLC**
**Des Plaines, IL 60016 (US)**
• **GISSELQUIST, Jana, L.**
**UOP LLC**
**Des Plaines, IL 60016 (US)**
• **PATTON, R., Lyle**
**UOP LLC**
**Des Plaines, IL 60016 (US)**
• **WILSON, Stephen, T.**
**UOP LLC**
**Des Plaines, IL 60016 (US)**
• **JAN, Deng-Yang**
**UOP LLC**
**Des Plaines, IL 60016-6101 (US)**
• **KOSTER, Susan, C.**
**UOP LLC**
**Des Plaines, IL 60016 (US)**

(74) Representative: **Chung, Hsu Min**
**Boult Wade Tennant**
**Verulam Gardens**
**70 Gray's Inn Road**
**London WC1X 8BT (GB)**

(56) References cited:
**US-A- 5 362 697        US-A1- 2003 211 034**
**US-B1- 6 613 302       US-B1- 6 713 041**

**Description**

BACKGROUND OF THE INVENTION

This invention relates to aluminosilicate zeolites designated UZM-8 and UZM-8HS and various uses thereof. These zeolites can be used as catalysts in processes such as xylene isomerization and ethylbenzene synthesis.

Zeolites are crystalline aluminosilicate compositions which are microporous and which are formed from corner sharing $AlO_2$ and $SiO_2$ tetrahedra. Numerous zeolites, both naturally occurring and synthetically prepared are used in various industrial processes. Synthetic zeolites are prepared via hydrothermal synthesis employing suitable sources of Si, Al, as well as structure directing agents such as alkali metals, alkaline earth metals, amines, or organoammonium cations. The structure directing agents reside in the pores of the zeolite and are largely responsible for the particular structure that is ultimately formed. These species balance the framework charge associated with aluminum and can also serve as space fillers. Zeolites are characterized by having pore openings of uniform dimensions, having a significant ion exchange capacity, and being capable of reversibly desorbing an adsorbed phase which is dispersed throughout the internal voids of the crystal without significantly displacing any atoms which make up the permanent zeolite crystal structure. Zeolites can be used as catalysts for hydrocarbon conversions, which can take place on outside surfaces as well as on internal surfaces within the pore.

Applicants have synthesized a new family of materials designated UZM-8. The UZM-8 compositions are aluminosilicates having Si/Al molar ratio from 6.5 to 35. The UZM-8 compositions show unique x-ray diffraction patterns compared to other known zeolites. These UZM-8 compositions are prepared from aqueous reaction mixtures containing either organoammonium compounds or a mixture of organoammonium compounds and alkali and/or alkaline earth compounds. The organoammonium compounds used to make UZM-8 are non-cyclic nor contain cyclic substituents and are generally quite simple. Preferred examples of organoammonium compounds used to make UZM-8 include the diethyldimethylammonium (DEDMA), ethyltrimethylammonium (ETMA) or hexamethonium (HM) cations.

Although UZM-8 compositions have some similarities to a layered material identified as MCM-56, there are sufficient differences that UZM-8 compositions are structurally different from MCM-56 materials and thus are unique new zeolites structures. The preparation of MCM-56 is disclosed in US-A-5,362,697 where it is stated that MCM-56 is prepared from a reaction mixture containing a combination of alkali metals and hexamethylene imine (HMI) as directing agents and requires that the silica source be a predominately solid silica source comprising at least 30 wt.% $SiO_2$. It is further stated in the '697 patent that the reaction must be stopped and quenched at a time before significant amounts of MCM-49 form in the reaction mixture. The synthesis of MCM-49 is disclosed in US-A-5,236,575 and again involves a combination of alkali metals and HMI structure directing agents plus a predominately solid silica source comprising at least 30 wt.% $SiO_2$. Upon calcination the MCM-49 composition is not readily distinguishable from calcined MCM-22 which has the MWW framework topology. It is further stated in J. Phys. Chem., 1996, 100, p.3788-3798, that in the as-synthesized form MCM-49 has essentially the MWW topology. Thus, MCM-56 is an intermediate structure in the formation of MCM-49 which in the calcined form is virtually the same as MCM-22 both of which have the MWW structure. The '697 patent further describes the MCM-56 as a layered structure in both its as-synthesized and calcined forms based on the claimed swellability of the material.

In contrast to MCM-56, UZM-8 is not an intermediate in the formation of MCM-49. Additionally, the UZM-8 materials can be synthesized from an alkali free reaction mixture using an organoammonium cation such as DEDMA cation that offers great stability and robustness without the formation of MCM-49 or other impurities. However, in the HMI/Na system, varying the relative amount of amine structure directing agent to alkali metal and/or alkaline earth metal compound can yield either the MCM-56/MCM-49 system with higher relative alkali content or a precursor to MCM-22 with lower relative alkali content. Reaction conditions, mainly temperature and time, are used to distinguish MCM-56 and MCM-49 in the higher alkali content system that is difficult to control, leading to the requirement for quenching the MCM-56 reaction mixture before significant amounts of MCM-49 form. Finally, UZM-8 is a layered material in that the as-synthesized form is swellable and has a x-ray diffraction pattern that is distinguishable from MCM-56.

The UZM-8 compositions have also been modified by using one or more techniques selected from acid extraction, calcination, steaming and ammonium hexafluorosilicate treatment, applicants have been able to control the aluminum content of the UZM-8 zeolites to nearly all silica while maintaining their structure and porosity. Dealumination strategies are known in the art and are given by Breck (see D. W. Breck, Zeolite Molecular Sieves, Wiley and Sons, New York, (1974), p. 441) and Skeels and Breck (see US-A-4,610,856). The result is a modified UZM-8 (UZM-8HS) material containing less aluminum than the starting UZM-8 composition. Control of the Al content in the zeolite allows one to tune the properties associated with the Al, such as ion-exchange capacity and acidity thereby providing improved catalysts and/or adsorbents. These modified compositions have been designated UZM-8HS.

The UZM-8 materials of this invention are thermally stable and can be used in their acidic forms as catalysts in hydrocarbon conversion processes, including ethylbenzene synthesis and xylene isomerization, but also in separation processes, adsorption, and ion-exchange applications.

BRIEF DESCRIPTION OF THE DRAWINGS

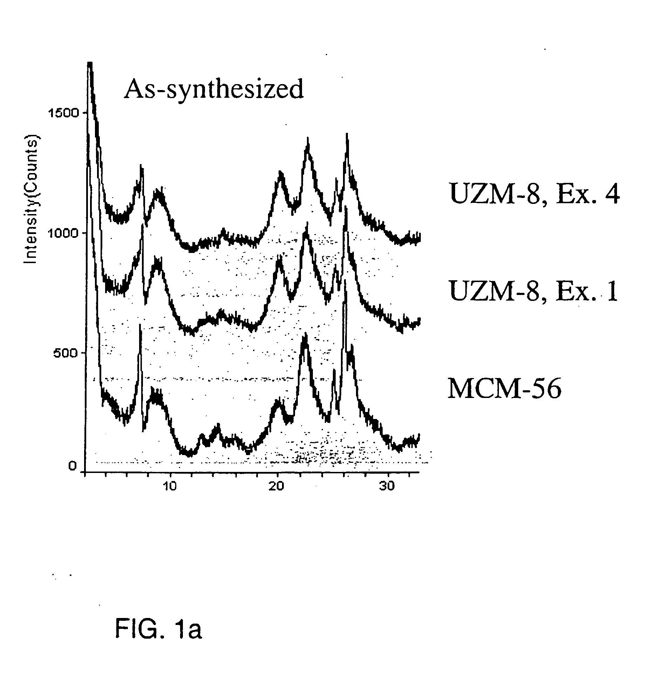

Figure 1a presents x-ray diffraction patterns for as-synthesized UZM-8 compositions of examples 1 and 4, and for zeolite MCM-56.

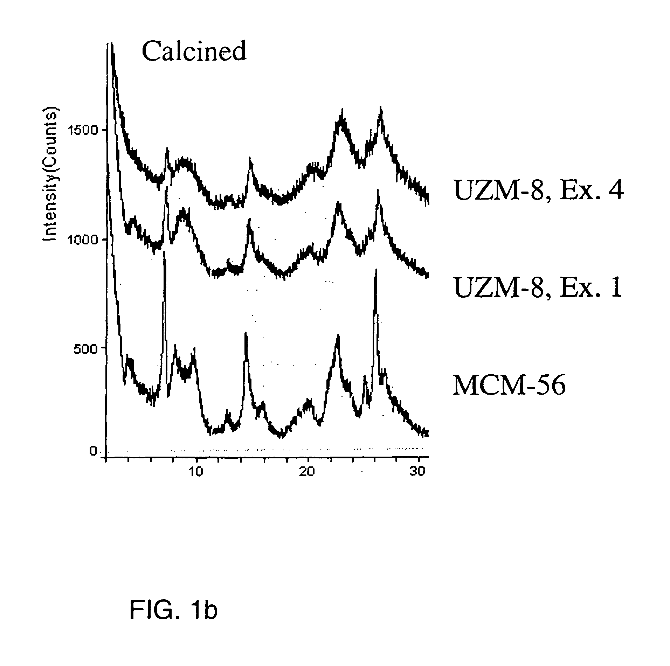

Figure 1b presents x-ray diffraction patterns for calcined UZM-8 compositions of examples 1 and 4, and for zeolite MCM-56.

DETAILED DESCRIPTION OF THE INVENTION

Applicants have prepared a family of aluminosilicate and substituted aluminosilicate zeolites which are designated UZM-8. In one embodiment of the invention, UZM-8 zeolites are prepared in an alkali-free reaction medium in which only one or more organoammonium species are used as structure directing agents. In this case, the microporous crystalline zeolite (UZM-8) has a composition in the as-synthesized form and on an anhydrous basis expressed by the empirical formula:

$$R_r^{p+}Al_{1-x}E_xSi_yO_z$$

where R is at least one organoammonium cation selected from the group consisting of protonated amines, protonated diamines, quaternary ammonium ions, diquaternary ammonium ions, protonated alkanolamines and quaternized alkanolammonium ions. Preferred organoammonium cations are those that are non-cyclic or those that that do not contain a cyclic group as one substituent. Of these those that contain at least two methyl groups as substituents are especially preferred. Examples of preferred cations include without limitation DEDMA, ETMA, HM and mixtures thereof. The ratio of R to (Al + E) is represented by "r" which varies from 0.05 to 5. The value of "p" which is the weighted average valence of R varies from 1 to 2. The ratio of Si to (Al + E) is represented by "y" which varies from 6.5 to 35. E is an element which is tetrahedrally coordinated, is present in the framework and is selected from the group consisting of gallium, iron, chromium, indium and boron. The mole fraction of E is represented by "x" and has a value from 0 to 0.5, while "z" is the mole ratio of O to (Al + E) and is given by the equation

$$z = (r \bullet p + 3 + 4 \bullet y)/2.$$

In another embodiment of the invention, the UZM-8 zeolites can be prepared using both organoammonium cations and alkali and/or alkaline earth cations as structure directing agents. As in the alkali-free case above, the same organoammonium cations can be used here. Alkali or alkaline earth cations are observed to speed up the crystallization of UZM-8, often when present in amounts less than 0.05 M$^+$/Si. For the alkali and/or alkaline earth metal containing systems, the microporous crystalline zeolite (UZM-8) has a composition in the as-synthesized form and on an anhydrous basis expressed by the empirical formula:

$$M_m^{n+}R_r^{p+}Al_{1-x}E_xSi_yO_z$$

where M is at least one exchangeable cation and is selected from the group consisting of alkali and alkaline earth metals. Specific examples of the M cations include but are not limited to lithium, sodium, potassium, rubidium, cesium, calcium, strontium, barium and mixtures thereof. Preferred R cations include without limitation DEDMA, ETMA, HM and mixtures thereof. The value of "m" which is the ratio of M to (Al + E) varies from 0.01 to 2. The value of "n" which is the weighted average valence of M varies from 1 to 2. The ratio of R to (Al + E) is represented by "r" which varies from 0.05 to 5. The value of "p" which is the weighted average valence of R varies from 1 to 2. The ratio of Si to (Al + E) is represented by "y" which varies from 6.5 to 35. E is an element which is tetrahedrally coordinated, is present in the framework and is selected from the group consisting of gallium, iron, chromium, indium and boron. The mole fraction of E is represented by "x" and has a value from 0 to 0.5, while "z" is the mole ratio of O to (Al + E) and is given by the equation

$$z = (m \bullet n + r \bullet p + 3 + 4 \bullet y)/2$$

where M is only one metal, then the weighted average valence is the valence of that one metal, i.e. +1 or +2. However, when more than one M metal is present, the total amount of

$$M^{n+}_m = M^{(n1)+}_{m1} + M^{(n2)+}_{m2} + M^{(n3)+}_{m3} + \ldots$$

and the weighted average valence "n" is given by the equation:

$$n = \frac{m_1 \bullet n_1 + m_2 \bullet n_2 + m_3 \bullet n_3 + \cdots}{m_1 + m_2 + m_3 \cdots}$$

Similarly when only one R organic cation is present, the weighted average valence is the valence of the single R cation, i.e., +1 or +2. When more than one R cation is present, the total amount of R is given by the equation.

$$R^{p+}_r = R^{(p1)+}_{r1} + R^{(p2)+}_{r2} + R^{(p3)+}_{r3}$$

and the weighted average valence "p" is given by the equation:

$$p = \frac{p_1 \bullet r_1 + p_2 \bullet r_2 + p_3 \bullet r_3 + \cdots}{r_1 + r_2 + r_3 + \cdots}$$

The microporous crystalline zeolites of the present invention are prepared by a hydrothermal crystallization of a reaction mixture prepared by combining reactive sources of R, aluminum, silicon and optionally M and E. The sources of aluminum include but are not limited to aluminum alkoxides, precipitated aluminas, aluminum metal, sodium aluminate, organoammonium aluminates, aluminum salts and alumina sols. Specific examples of aluminum alkoxides include, but are not limited to aluminum ortho sec-butoxide and aluminum ortho isopropoxide. Sources of silica include but are not limited to tetraethylorthosilicate, colloidal silica, precipitated silica, alkali silicates and organoammonium silicates. A special reagent consisting of an organoammonium aluminosilicate solution can also serve as the simultaneous source of Al, Si, and R. Sources of the E elements include but are not limited to alkali borates, boric acid, precipitated gallium oxyhydroxide, gallium sulfate, ferric sulfate, ferric chloride, chromium nitrate and indium chloride. Sources of the M metals include the halide salts, nitrate salts, acetate salts, and hydroxides of the respective alkali or alkaline earth metals. R can be introduced as an organoammonium cation or an amine. When R is a quaternary ammonium cation or a quaternized alkanolammonium cation, the sources include but are not limited the hydroxide, chloride, bromide, iodide and fluoride compounds. Specific examples include without limitation DEDMA hydroxide, ETMA hydroxide, tetramethylammonium hydroxide, tetraethylammonium hydroxide, hexamethonium bromide, tetrapropylammonium hydroxide, methyltriethylammonium hydroxide, tetramethylammonium chloride and choline chloride. R may also be introduced as an amine, diamine, or alkanolamine that subsequently hydrolyzes to form an organoammonium cation. Specific non-limiting examples are N,N,N',N'-tetramethyl -1,6-hexanediamine, triethylamine, and triethanolamine. Preferred sources of R without limitation are ETMAOH, DEDMAOH, and $HM(OH)_2$.

The reaction mixture containing reactive sources of the desired components can be described in terms of molar ratios of the oxides by the formula:

$$aM_{2/n}O : bR_{2/p}O : 1\text{-}cAl_2O_3 : cE_2O_3 : dSiO_2 : eH_2O$$

where "a" varies from 0 to 25, "b" varies from 1.5 to 80, "c" varies from 0 to 1.0, "d" varies from 10 to 100, and "e" varies from 100 to 15000. If alkoxides are used, it is preferred to include a distillation or evaporative step to remove the alcohol hydrolysis products. The reaction mixture is now reacted at a temperature of 85°C to 225°C and preferably from 125°C to 150°C for a period of 1 day to 28 days and preferably for a time of 5 days to 14 days in a sealed reaction vessel under autogenous pressure. After crystallization is complete, the solid product is isolated from the heterogeneous mixture by means such as filtration or centrifugation, and then washed with deionized water and dried in air at ambient temperature up to 100°C

The UZM-8 aluminosilicate zeolite, which is obtained from the above-described process, is characterized by an x-ray diffraction pattern, having at least the *d*-spacings and relative intensities set forth in Table A below

Table A

| *d*-Spacings and Relative Intensities for as-synthesized UZM-8 | | |
|---|---|---|
| 2-θ | d(Å) | I/I$_0$% |
| 6.40 - 6.90 | 13.80 - 12.80 | w - s |
| 6.95 - 7.42 | 12.70 - 11.90 | m-s |
| 8.33-9.11 | 10.60 - 9.70 | w-vs |
| 19.62 - 20.49 | 4.52 - 4.33 | m - vs |
| 21.93 - 22.84 | 4.05 - 3.89 | m - vs |
| 24.71 - 25.35 | 3.60 - 3.51 | w - m |
| 25.73 - 26.35 | 3.46 - 3.38 | m - vs |

The UZM-8 compositions are stable to at least 600°C and usually at least 700°C. The characteristic diffraction lines associated with typical calcined UZM-8 samples are shown below in table B. The as-synthesized form of UZM-8 is expandable with organic cations, indicating a layered structure.

Table B

| *d*-Spacings and Relative Intensity for Calcined UZM-8 | | |
|---|---|---|
| 2-θ | d(Å) | I/I$_0$% |
| 4.05 - 4.60 | 21.80 - 19.19 | w - m |
| 7.00 - 7.55 | 12.62 - 11.70 | m - vs |
| 8.55 - 9.15 | 10.33 - 9.66 | w - vs |
| 12.55 - 13.15 | 7.05 - 6.73 | w |
| 14.30 - 14.90 | 6.19 - 5.94 | m - vs |
| 19.55 - 20.35 | 4.54 - 4.36 | w - m |
| 22.35 - 23.10 | 3.97 - 3.85 | m - vs |
| 24.95 - 25.85 | 3.57 - 3.44 | w - m |
| 25.95 - 26.75 | 3.43 - 3.33 | m - s |

An aspect of the UZM-8 synthesis that contributes to some of its unique properties is that it can be synthesized from a homogenous solution. In this chemistry, soluble aluminosilicate precursors condense during digestion to form extremely small crystallites that have a great deal of external surface area and short diffusion paths within the pores of the crystallites. This can affect both adsorption and catalytic properties of the material.

As-synthesized, the UZM-8 material will contain some of the charge balancing cations in its pores. In the case of syntheses from alkali or alkaline earth metal-containing reaction mixtures, some of these cations may be exchangeable cations that can be exchanged for other cations. In the case of organoammonium cations, they can be removed by heating under controlled conditions. In the cases where UZM-8 is prepared in an alkali-free system, the organoammonium cations are best removed by controlled calcination, thus generating the acid form of the zeolite without any intervening ion-exchange steps. On the other hand, it may sometimes be possible to remove a portion of the organoammonium via ion exchange. In a special case of ion exchange, the ammonium form of UZM-8 may be generated via calcination of the organoammonium form of UZM-8 in an ammonia atmosphere.

The properties of the UZM-8 compositions described above can be modified by removing some of the aluminum atoms from the framework and optionally inserting silicon atoms. Treating processes include without limitation treatment with a fluorosilicate solution or slurry, extraction with a weak, strong or complexing acid, etc. In carrying out these dealumination treatments the particular form of the UZM-8 is not critical, but can have a bearing on the final product especially with regard to the extent of dealumination. Thus, the UZM-8 can be used as synthesized or can be ion exchanged to provide a different cation form. In this respect, the starting zeolite can be described by the empirical formula:

$$M_{m'}^{n'+} R_{r'}^{p+} Al_{(1-x)} E_x Si_y O_{z'}$$   (2)

where R, x, y, and E are as described above and m' has a value from 0 to 7.0, M' is a cation selected from the group consisting of alkali metals, alkaline earth metals, rare earth metals, hydrogen ion, ammonium ion, and mixtures thereof, n' is the weighted average valence of M' and varies from 1 to 3, r' has a value from 0 to 7.0, r' + m' > 0, and p is the weighted average valence of R and varies from +1 to +2. The value of z' is given by the formula:

$$z' = (m' \bullet n' + r' \bullet p + 3 + 4 \bullet y)/2.$$

The designation UZM-8 will be used to refer to the zeolite represented by formula (2) which includes both the as-synthesized and ion exchanged forms of the zeolite. The modified compositions will be referred to as UZM-8HS.

Methods used to exchange one cation for another are well known in the art and involve contacting the microporous compositions with a solution containing the desired cation (at molar excess) at exchange conditions. Exchange conditions include a temperature of 15°C to 100°C and a time of 20 minutes to 50 hours. The organic cation can also be removed prior to ion exchange by heating under controlled conditions. A special case of ion-exchange is ammonia calcination, in which the organic template can be decomposed and replaced by ammonium cation.

In a preferred case, especially for dealumination by treatment with a fluorosilicate solution, the UZM-8 is exchanged with ammonium cation by contacting it with ammonium nitrate at a temperature of 15°C to 100°C, followed by a water wash. This procedure may be repeated several times. Finally, the exchanged UZM-8 zeolite is dried at 100°C.

One process of preparing the UZM-8HS of the present invention is by treating the UZM-8 composition described above with a fluorosilicate salt at a temperature of 20°C to 90°C. The fluorosilicate salt serves two purposes. It removes aluminum atoms from the framework and provides a source of extraneous silicon, which can be inserted into the framework (replacing the aluminum). The fluorosilicate salts which can be used are described by the general formula:

$$A_{2/n}SiF_6$$

where n is the valence of A and A is a cation selected from the group consisting of $NH_4^+$, $H^+$, $Mg^{2+}$, $Li^+$, $Na^+$, $Ba^{2+}$, $Cd^{2+}$, $Cu^+$, $Cu^{2+}$, $Ca^{2+}$, $Cs^+$, $Fe^{2+}$, $Ca^{2+}$, $Pb^{2+}$, $Mn^{2+}$, $Rb^+$, $Ag^+$, $Sr^{2+}$, $Tl^+$, and $Zn^{2+}$. The ammonium fluorosilicate is most preferred because of its substantial solubility in water and because it forms water soluble by-product salts upon reaction with the zeolite, namely $(NH_4)_3AlF_6$.

The fluorosilicate salt is contacted with the UZM-8 zeolite in the form of an aqueous solution or slurry at a pH in the range of 3 to 7. This solution is contacted with the zeolite either incrementally or continuously at a slow rate such that a sufficient proportion of the framework aluminum atoms removed are replaced by silicon atoms to retain at least 50%, preferably at least 70% of the framework (crystalline) structure of the starting UZM-8 zeolite. The amount of fluorosilicate necessary to carry out the process of this invention can vary considerably, but should be at least in an amount of 0.0075 moles of the fluorosilicate salt per 100 grams of starting zeolite. Once the reaction is complete, the product zeolite UZM-8HS is isolated by conventional techniques such as filtration.

Without wishing to be bound by any particular theory, the process of removing aluminum and inserting the silicon appears to proceed in two steps in which the aluminum extraction step will, unless controlled, proceed very rapidly while the silicon insertion is relatively slow. If dealumination becomes too extensive without silicon substitution, the crystal structure becomes seriously degraded and ultimately collapses. In general, the rate of aluminum extraction is decreased as the pH of the fluorosilicate solution in contact with the zeolite is increased within the range of 3 to 7 and as the concentration of the fluorosilicate in the reaction system is decreased. At pH values below 3, crystal degradation can be unduly severe, whereas at pH values higher than 7, silicon insertion is unduly slow. Also, increasing the reaction temperature tends to increase the rate of substitution of silicon. Increasing the reaction temperature has been found to have less of an effect on dealumination than the pH of the solution. Therefore, the pH may be considered a means of controlling the dealumination while temperature may be considered as a means of controlling the substitution rate.

Theoretically, there is no lower limit for the concentration of fluorosilicate salt in the aqueous solution employed, provided, of course, the pH of the solution is high enough to avoid undue destructive attack on the UZM-8 zeolite structure apart from the intended reaction with the fluorosilicate. A slow rate of addition of fluorosilicate salts insures that adequate time is permitted for the inserton of silicon into the framework before excessive aluminum extraction occurs with consequent collapse of the crystal structure. In general the effective reaction temperature is between 10°C and 99°C, preferably between 20°C and 95°C, but temperatures of 125°C or higher and as low as 0°C can be used.

The maximum concentration of fluorosilicate salt in the aqueous solution employed is, of course, interrelated to the temperature and pH factors and also with the time of contact between the zeolite and the solution and the relative proportions of zeolite and fluorosilicate salt. Solutions having fluorosilicate salt concentrations of between $10^{-3}$ moles per liter of solution and up to saturation of the solution can be employed, but it is preferred that concentrations in the range of between 0.05 and 2.0 moles per liter of solution be used. In addition, as hereinbefore discussed, slurries of the fluorosilicate salts may be employed. The aforementioned concentration values are with respect to true solutions, and are not intended to apply to the total fluorosilicate salts in slurries of the salts in water. Even very slightly soluble fluorosilicate salts can be slurried in water and used as a reagent, the undissolved solids being readily available to replace dissolved molecular species consumed in reaction with the zeolite. The minimum value for the amount of fluoro salt to be added is preferably at least equivalent to the minimum mole fraction of aluminum to be removed from the zeolite.

It has been found that when large amounts of silicon atoms are to be substituted, i.e., increasing the $SiO_2/Al_2O_3$ ratio by more than 100%, it is preferable to carry out the process in multiple steps in order to minimize crystal degradation. As the amount of silicon that is substituted into the framework is substantially increased (beyond 100% increase) it may actually be necessary to carry out the process in two or more steps in order to prevent excessive degradation of the crystalline structure. That is, contacting with the fluorosilicate salt is carried out in two or more steps using a lower concentration of the fluorosilicate salt than required to replace the desired amount of silicon in one step. After each fluorosilicate treatment, the product is washed to remove fluoride and aluminum. Drying of the zeolite at 50°C between treatments may also be done to facilitate the handling of the wet zeolite product.

Another treatment method involves contacting the UZM-8 starting zeolite with an acid (acid extraction) in order to remove some of the aluminum from the framework and thereby provide the UZM-8HS. Although it is known that aluminum can be extracted from the framework by acids, it is not predictable whether the resulting product will retain a substantial portion of its crystallinity or whether the structure will collapse resulting in an amorphous material. Applicants have discovered that UZM-8 can be dealuminated to nearly pure silica forms while maintaining substantial crystallinity, surface area and micropore volume.

The acids which can be used in carrying out acid extraction include without limitation mineral acids, carboxylic acids and mixtures thereof. Examples of these include sulfuric acid, nitric acid, ethylene diaminetetraacetic acid (EDTA), citric acid, oxalic acid, etc. The concentration of acid which can be used is not critical but is conveniently between 1 wt. % to 80 wt.% acid and preferably between 5 wt.% and 40 wt.% acid. Acid extraction conditions include a temperature of 10°C to 100°C for a time of 10 minutes to 24 hours. Once treated with the acid, the UZM-8HS zeolite is isolated by means such as filtration, washed with deionized water and dried at ambient temperature up to 100°C.

The extent of dealumination obtained from acid extraction depends on the cation form of the starting UZM-8 as well as the acid concentration and the time and temperature over which the extraction is conducted. For example, if organic cations are present in the starting UZM-8, the extent of dealumination will be slight compared to a UZM-8 in which the organic cations have been removed. This may be preferred if it is desired to have dealumination just at the surface of the UZM-8. Convenient ways of removing the organic cations include calcination, ammonia calcination, steaming and ion exchange. Calcination conditions include a temperature of 300°C to 600°C for a time of 2 to 24 hours. Steaming conditions include a temperature of 400°C to 850°C with from 1 % to 100% steam for a time of 10 minutes to 48 hours and preferably a temperature of 500°C to 600°C, steam concentration of 5 to 50% and a time of 1 to 2 hours. Ion exchange conditions are as set forth above.

A special treatment for removing organic cations to obtain the ammonium ion exchanged form is ammonia calcination. Calcination in an ammonia atmosphere can decompose organic cations, presumably to a proton form that can be neutralized by ammonia to form the ammonium cation. The stability of the ammonium form of the zeolite prevents dealumination upon hydration, which occurs extensively in, lower ratio zeolites in the proton forms obtained in air calcinations. The resulting ammonium form of the zeolite can be further ion-exchanged to any other desired form. Ammonia calcination conditions include treatment in the ammonia atmosphere at temperatures between 250°C and 600°C and more preferably between 250°C and 450°C for times of 10 minutes to 5 hours. Optionally, the treatments can be carried out in multiple steps within this temperature range such that the total time in the ammonia atmosphere does not exceed 5 hours. Above 500°C, the treatments should be brief, less than a half hour and more preferably on the order of 5-10 minutes. Extended calcination times above 500°C can lead to unintended dealumination along with the desired ammonium ion-exchange and are unnecessarily harsh as most organoammonium templates easily decompose at lower temperatures.

It should be pointed out that both calcination and steaming treatments not only remove organic cations, but can also dealuminate the zeolite. Thus, alternate embodiments of the invention include: a calcination treatment followed by acid extraction and steaming followed by acid extraction. A further embodiment of the invention comprises calcining or steaming the starting UZM-8 zeolite followed by an ion-exchange treatment. Of course an acid extraction can be carried out concurrently with, before or after the ion exchange.

The ion exchange conditions are the same as set forth above, namely a temperature of 15°C to 100°C and a time of 20 minutes to 50 hours. Ion exchange can be carried out with a solution comprising a cation (M1') selected from

7

the group consisting of alkali metals, alkaline earth metals, rare earth metals, hydrogen ion, ammonium ion, and mixtures thereof. By carrying out this ion exchange, the M1 cation is exchanged for a secondary or different M1' cation. In a preferred embodiment, the UZM-8HS composition after the steaming or calcining steps is contacted with an ion exchange solution comprising an ammonium salt. Examples of ammonium salts include but are not limited to ammonium nitrate, ammonium chloride, ammonium bromide, and ammonium acetate. The ammonium ion containing solution can optionally contain a mineral acid such as but not limited to nitric, hydrochloric, sulfuric and mixtures thereof. The concentration of the mineral acid is that amount necessary to give a ratio of $H^+$ to $NH_4^+$ of 0 to 1. This ammonium ion exchange aids in removing any debris present in the pores after the steaming and/or calcination treatments.

It is apparent from the foregoing that, with respect to effective process conditions, it is desirable that the integrity of the zeolite crystal structure be substantially maintained throughout the dealumination process, and that the zeolite retains at least 50%, preferably at least 70 and more preferably at least 90% of its original crystallinity. A convenient technique for assessing the crystallinity of the products relative to the crystallinity of the starting material is the comparison of the relative intensities of the *d*-spacing of their respective X-ray powder diffraction patterns. The sum of the peak intensities, in arbitrary units above the background, of the starting material is used as the standard and is compared with the corresponding peak intensities of the products. When, for example, the numerical sum of the peak heights of the molecular sieve product is 85 percent of the value of the sum of the peak intensities of the starting zeolite, then 85 percent of the crystallinity has been retained. In practice it is common to utilize only a portion of the peaks for this purpose, as for example, five or six of the strongest peaks. Other indications of the retention of crystallinity are surface area and adsorption capacity. These tests may be preferred when the substituted metal significantly changes, e.g., increases, the absorption of x-rays by the sample or when peaks experience substantial shifts such as in the dealumination process.

After having undergone any of the dealumination treatments as described above, the UZM-8HS is usually dried and can be used in various processes as discussed below. Applicants have found the properties of the UZM-8HS can be further modified by one or more additional treatment. These treatments include steaming, calcining or ion exchanging and can be carried out individually or in any combination. Some of these combinations include but are not limited to:

| steam calcine | | ion exchange; | | |
| calcine | $\rightarrow$ | steam | $\rightarrow$ | ion exchange; |
| ion exchange | $\rightarrow$ | calcine | $\rightarrow$ | steam |
| ion exchange | $\rightarrow$ | steam | $\rightarrow$ | calcine; |
| | steam | $\rightarrow$ | calcine; | |
| | $\rightarrow$ | | | |
| | calcine | | steam, etc. | |

The dealumination treatment described above can be combined in any order to provide the zeolites of the invention although not necessarily with equivalent result. It should be pointed out that the particular sequence of treatments, e.g., AFS, acid extraction, steaming, calcining, etc can be repeated as many times as necessary to obtain the desired properties. Of course one treatment can be repeated while not repeating other treatments, e.g., repeating the AFS two or more times before carrying out steaming or calcining; etc. Finally, the sequence and/or repetition of treatments will determine the properties of the final UZM-8HS composition.

The UZM-8HS as prepared above is described by the empirical formula on an anhydrous basis of:

$$M1_a^{n+}Al_{(1-x)}E_xSi_{y'}O_{z''}$$

where M1 is at least one exchangeable cation selected from the group consisting of alkali, alkaline earth metals, rare earth metals, ammonium ion, hydrogen ion and mixtures thereof, **a** is the mole ratio of M1 to (Al + E) and varies from 0.05 to 5.0, **n** is the weighted average valence of M1 and has a value of +1 to +3, E is an element selected from the group consisting of gallium, iron, boron, chromium, indium and mixtures thereof, **x** is the mole fraction of E and varies from 0 to 1.0, **y'** is the mole ratio of Si to (Al + E) and varies from greater than 6.5 to virtually pure silica and **z''** is the mole ratio of O to (Al + E) and has a value determined by the equation:

$$z'' = (a \bullet n + 3 + 4 \bullet y')/2.$$

By virtually pure silica is meant that virtually all the aluminum and/or the E metals have been removed from the framework. It is well know that it is virtually impossible to remove all the aluminum and/or E metal. Numerically, a zeolite is virtually pure silica when **y'** has a value of at least 3,000, preferably 10,000 and most preferably 20,000. Thus, ranges for **y'** are from 6.5 to 3,000 preferably greater than 10 to 3,000; 6.5 to 10,000 preferably greater than 10 to 10,000 and 6.5 to 20,000 preferably greater than 10 to 20,000.

In specifying the proportions of the zeolite starting material or adsorption properties of the zeolite product and the like herein, the "anhydrous state" of the zeolite will be intended unless otherwise stated. The term "anhydrous state" is employed herein to refer to a zeolite substantially devoid of both physically adsorbed and chemically adsorbed water.

The UZM-8HS zeolite obtained after one or more of the above described treatments will have x-ray diffraction patterns which are different (and thus unique) from that of UZM-8. A list of the major peaks that are common to all the UZM-8HS materials is given in Table C.

Table C

| UZM-8HS | | |
|---|---|---|
| 2-θ | d(Å) | $I/I_o$% |
| 6.90 - 7.40 | 12.80 - 11.94 | w-vs |
| 8.15 - 8.85 | 10.84 - 9.98 | m-vs |
| 14.10 - 14.70 | 6.28 - 6.02 | w-vs |
| 19.40 - 20.10 | 4.57 - 4.41 | w-s |
| 22.00 - 22.85 | 4.04 - 3.89 | m-vs |
| 24.65 - 25.40 | 3.61 - 3.50 | w-m |
| 25.70 - 26.50 | 3.46 - 3.36 | w-vs |

The zeolites of this invention are capable of separating mixtures of molecular species based on the molecular size (kinetic diameter) or on the degree of polarity of the molecular species. When the separation of molecular species is based on molecular size, separation is accomplished by the smaller molecular species entering the intracrystalline void space while excluding larger species. The kinetic diameters of various molecules such as oxygen, nitrogen, carbon dioxide, carbon monoxide and various hydrocarbons are provided in D.W. Breck, Zeolite Molecular Sieves, John Wiley and Sons (1974) p. 636. The separation of hydrocarbons based on molecular size is a preferred application.

The UZM-8 and UZM-8HS crystalline microporous compositions of the present invention in any of the forms described above can be used as catalysts or catalyst supports in hydrocarbon conversion processes. Hydrocarbon conversion processes are well known in the art and include cracking, hydrocracking, alkylation of both aromatics and isoparaffins, isomerization, polymerization, reforming, dewaxing, hydrogenation, dehydrogenation, transalkylation, dealkylation, hydration, dehydration, hydrotreating, hydrodenitrogenation, hydrodesulfurization, methanation and syngas shift process. Preferred hydrocarbon conversion processes are alkylation of aromatics, isomerization of xylenes, naphtha cracking, ring opening and conversion of oxygenates to olefins.

Other reactions may be catalyzed by these crystalline microporous compositions, including base-catalyzed side chain alkylation of alkylaromatics, aldol-condensations, olefin double bond isomerization and isomerization of acetylenes, alcohol dehydrogenation, and olefin dimerization and oligomerization. Some of the reaction conditions and types of feeds that can be used in these processes are set forth in US-A-5,015,796 and in H. Pines, THE CHEMISTRY OF CATALYTIC HYDROCARBON CONVERSIONS, Academic Press (1981) pp. 123-154 and references contained therein.

Hydrocracking conditions typically include a temperature in the range of 204 to 649°C, preferably between 316 to 510°C. Reaction pressures are in the range of atmospheric to 24,132 kPa g, preferably between 1379 - 20,685 kPa g. Contact times usually correspond to liquid hourly space velocities (LHSV) in the range of 0.1 hr$^{-1}$ to 15 hr$^{-1}$ preferably between 0.2 and 3 hr$^{-1}$. Hydrogen circulation rates are in the range of 178-8,888 std. m$^3$/m$^3$, preferably between 355-5,333 std. m$^3$/m$^3$. Suitable hydrotreating conditions are generally within the broad ranges of hydrocracking conditions set out above.

The reaction zone effluent is normally removed from the catalyst bed, subjected to partial condensation and vapor-liquid separation and then fractionated to recover the various components thereof. The hydrogen, and if desired some or all of the unconverted heavier materials, are recycled to the reactor. Alternatively, a two-stage flow may be employed with the unconverted material being passed into a second reactor. Catalysts of the subject invention may be used in just one stage of such a process or may be used in both reactor stages.

Catalytic cracking processes are preferably carried out with the UZM-8 composition using feedstocks such as gas oils, heavy naphthas, deasphalted crude oil residua, etc. with gasoline being the principal desired product. Temperature conditions of 454°C to 595°C, LHSV values of 0.5 to 10 hr $^{-1}$ and pressure conditions of from 0 kPag to 345 kPag are suitable.

Alkylation of aromatics usually involves reacting an aromatic ($C_2$ to $C_{12}$), especially benzene, with a monoolefin to produce a linear alkyl substituted aromatic. The process is carried out at an aromatic: olefin (e.g., benzene:olefin) ratio of between 5:1 and 30:1, a LHSV of 0.3 to 6 hr$^{-1}$, a temperature of 100° to 250°C and pressures of 1379 kPag to 6895 kPag. Further details on apparatus may be found in US-A-4,870,222 which is incorporated by reference.

Alkylation of isoparaffins with olefins to produce alkylates suitable as motor fuel components is carried out at temperatures of -30° to 40°C, pressures from atmospheric to 6,894 kPa and a weight hourly space velocity (WHSV) of 0.1 to 120. Details on paraffin alkylation may be found in US-A-5,157,196 and US-A-5,157,197, which are incorporated by reference.

The structures of the UZM-8 and UZM-8HS zeolites of this invention were determined by x-ray analysis. The x-ray patterns presented in the following examples were obtained using standard x-ray powder diffraction techniques. The radiation source was a high-intensity, x-ray tube operated at 45 kV and 35 ma. The diffraction pattern from the copper K-alpha radiation was obtained by appropriate computer based techniques. Flat compressed powder samples were either continuously scanned at 2° to 70° (2θ) or in a step mode from 4° to 35° (2θ). Interplanar spacings (d) in Angstrom units were obtained from the position of the diffraction peaks expressed as θ where θ is the Bragg angle as observed from digitized data. Intensities were determined from the integrated area of diffraction peaks after subtracting background, "$I_o$" being the intensity of the strongest line or peak, and "I" being the intensity of each of the other peaks.

As will be understood by those skilled in the art the determination of the parameter 2θ is subject to both human and mechanical error, which in combination can impose an uncertainty of $\pm 0.4°$ on each reported value of 2θ. This uncertainty is, of course, also manifested in the reported values of the *d*-spacings, which are calculated from the 2θ values. This imprecision is general throughout the art and is not sufficient to preclude the differentiation of the present crystalline materials from each other and from the compositions of the prior art. In some of the x-ray patterns reported, the relative intensities of the *d*-spacings are indicated by the notations vs, s, m, and w which represent very strong, strong, medium, and weak, respectively. In terms of 100% x $I/I_o$, the above designations are defined as:

w = 0-15; m = 15-60: s = 60-80 and vs = 80-100

In certain instances the purity of a synthesized product may be assessed with reference to its x-ray powder diffraction pattern. Thus, for example, if a sample is stated to be pure, it is intended only that the x-ray pattern of the sample is free of lines attributable to crystalline impurities, not that there are no amorphous materials present.

In order to more fully illustrate the invention, the following examples are set forth. It is to be understood that the examples are only by way of illustration and are not intended as an undue limitation on the broad scope of the invention as set forth in the appended claims.

EXAMPLES

The following abbreviations will be used in the examples:

| | |
|---|---|
| Al (Oi-Pr)$_3$ | — aluminum isopropoxide |
| Al (Osec-Bu)$_3$ | — aluminum tri-sec-butoxide |
| DEDMAOH | — diethyldimethylammonium hydroxide |
| ETMAOH | — ethyltrimethylammonium hydroxide |
| HM(OH)$_2$ | — hexamethonium dihydroxide |
| MTEAOH | — methyltriethylammonium hydroxide |
| TEAOH | — tetraethylammonium hydroxide |
| TEOS | — tetraethylorthosilicate |
| TMACl | — tetramethylammonium chloride |
| TPAOH | — tetrapropylammonium hydroxide |

EXAMPLE 1

An aluminosilicate solution, designated Reagent A, was prepared by mixing 4.63g, Al(Osec-Bu)$_3$ (97%), , and 159.63g DEDMAOH (20% aq) with vigorous mixing, and adding to it 189.78g TEOS (98%) and 145.96g deionized $H_2O$. The reaction mixture was homogenized for 2 hr, forming a homogenous aluminosilicate solution. This solution was then concentrated on a rotary evaporator, as the by-products of hydrolysis, ethanol and sec-butanol, and some water was removed. Elemental analysis of this aluminosilicate stock solution showed it to contain 7.86 wt.% Si and 0.16 wt.% Al.

Another aluminosilicate stock solution, designated Reagent B, was prepared by mixing 60.91 g, Al(Osec-Bu)$_3$ (97%), and 280.00g DEDMAOH with vigorous mixing, and adding to it 99.86g TEOS and 59.23g de-ionized H$_2$O. The reaction mixture was homogenized for 4.5 hr as above yielding a clear homogenous solution.

A 79.59g portion of Reagent A and a 15.63g portion of Reagent B were combined and to it there were added, with mixing, 29.80g DEDMAOH. The resulting solution was homogenized for 30 min transferred to 2 x 125 mL Teflon™-lined autoclaves which were placed in an oven set at 150°C and the solution digested for 14 days and 19 days. The solid products were collected by centrifugation, washed with de-ionized water and dried at 95°C.

Powder x-ray diffraction analysis identified both of the isolated products as the material designated UZM-8. Characteristic lines in the diffraction pattern are shown in Table 1 below. Analytical results for the 14 day sample are presented in Table 2. The BET surface area was found to be 472 m$^2$/g and the micropore volume was 0.11 cc/g.

EXAMPLES 2-13

A series of examples were carried out to prepare UZM-8 compositions using different templates, conditions and silicon sources. The general process involved forming a mixture of Al(Osec-Bu)$_3$ and an organic templating agent, e.g. DEDMAOH. To this there was added a silicon source and the mixture was homogenized. If the silicon source was TEOS, the solution was concentrated by removing the ethanol and sec-butanol and some water which formed as hydrolysis products of the alkaxides. Optionally the solution/mixture was aged followed by the addition of a second templating agent and finally crystallization. The solid product was collected, washed, dried and then characterized by several analytical methods including x-ray diffraction analysis. The specific conditions for each example are presented in Table 2 and the characterization data are presented in Tables 3-7

Table 2 - Reaction Mixture Compositions and Reaction Conditions

| | REACTION MIXTURE A | | | | REACTION MIXTURE B | | | |
|---|---|---|---|---|---|---|---|---|
| Example # | Al (Osec-Bu)$_3$ (g) | Silicon Source (g) | First Template | Aged °C-hr | Mixture A (g) | Second Template (g) | Crystallization | |
| | | | | | | | Time (day) | Temp (°C) |
| 2 | 28.03 | Collsidal silica 324.76 | DEDMAOH (644.36) | 100-67 | 60.47 | ——————— | 7 | 150 |
| 3 | 28.03 | Collsidal silica (324.76) | DEDMAOH (644.36) | 100-67 | 55.14 | TMACl (0.79) +H$_2$O (4.08) | 7 | 150 |
| 4 | 6.44 | TEOS (161.76) | DEDMAOH (151.18) | ——— | 43.69 | DEOMAOH (24.24) NaCl (0.5) + H$_2$O (1.56) | 7 | 150 |
| 5 | 66.51 | Precipitated silica (208.95) | DEDMAOH (918.29) | ——— | All of A* | Na2SO4 (37.2) + H$_2$O (169.05) | 10 | 150 |
| 6 | 17.28 | Colloidal silica (260.12) | ETMAOH (719.57) | 98-overnight | 154.21 | NaCl (3.67) + H$_2$O (52.11) | 7 | 150 |
| 7 | 8.11 | Precipitated silica (43.30) | DEDMAOH (133.22) | ——— | All of A | KCl (1.19) + TMACl (3.61) + H$_2$O (10.56) | 145 HRS | 150 |
| 8 | 28.03 | Colloidal silica (324.76) | DEDMAOH | 100-67 | 91.67 | TMACl (1.31) LiCl | 7 | 150 |

(continued)

| | REACTION MIXTURE A | | | | REACTION MIXTURE B | | | |
|---|---|---|---|---|---|---|---|---|
| Example # | Al (Osec-Bu)$_3$ (g) | Silicon Source (g) | First Template | Aged °C-hr | Mixture A (g) | Second Template (g) | Crystallization | |
| | | | | | | | Time (day) | Temp (°C) |
| 9 | 12.81 | Colloidal silica (189.37) | DEDMAOH (187.87) MTEAOH (209.98) | 95-66 | 113.32 | NaCl (2.87) + H$_2$O (8.80) TMACl (1.04) | 10 | 150 |
| 10 | 13.75 | Colloidal silica (159.31) | DEDMAOH (221.27) | 100-41 | 56.65 | CaCl (0.77) + H$_2$O (1.53) | 7 | 175 |
| 11 | 15.39 | Colloidal silica (235.93) | TPAOH (99.79) DEDMAOH (349.93) | 95-overnight | 159.39 | NaCl (4.15) + H$_2$O (16.50) | 14 | 125 |
| 12 | 15.16 | Colloidal silica (232.87) | DEDMAOH (396.02) TPAOH (56.43) | 95-overnight | 125.26 | NaCl (3.27) + H$_2$O (11.48) H$_2$O | 14 | 125 |
| 13 | 15.16 | Colloidal silica (232.87) | DEDMAOH (396.02) TPAOH (56.43) | 95-overnight | 102.89 | NaCl (2.67) H$_2$O (942) | 25 | 115 |

*1.7 g of UZM-8 seeds also added

Table 3

| Analytical Results For Various UZM-8 Compositions | | | | | | |
|---|---|---|---|---|---|---|
| Example# | Si/Al | M/Al | N/Al | C/N | Surface* area (m$^2$/g) | Pore Volume* cc/g |
| 1 | 15.23 | | 1.55 | 5.44 | 472 | 0.11 |
| 2 | 16.18 | | 2.14 | 5.62 | 370 | 0.12 |
| 3 | 14.38 | M=Na 0.02 | 2.02 | 5.58 | 370 | 0.12 |
| 4 | 23.91 | M=Na 0.42 | 3.16 | 5.46 | 356 | 0.12 |
| 5 | 9.96 | M=Na 0.26 | 1.23 | 4.83 | 343 | 0.14 |
| 6 | 12.97 | M=Na 0.37 | 1.77 | 4.92 | 360 | 0.14 |
| 7 | 16.34 | M=K 0.14 | 1.88 | | 283 | 0.08 |
| 8 | 16.22 | M=Li 0.31 | 3.52 | 5.54 | 403 | 0.15 |
| 9 | 17.63 | M=Na 1.82 | 2.86 | 7.19 | 328 | 0.12 |

(continued)

| Analytical Results For Various UZM-8 Compositions | | | | | | |
|---|---|---|---|---|---|---|
| Example# | Si/Al | M/Al | N/Al | C/N | Surface* area (m²/g) | Pore Volume* cc/g |
| 10 | 18.2 | M=Cs 0.35 | 2.48 | 4.98 | | |
| 11 | 12.57 | M=Na 0.37 | 1.79 | 7.37 | 391 | 0.11 |
| 12 | 13.60 | M=Na 0.80 | 3.02 | 7.82 | 312 | 0.10 |
| 13 | 11.31 | M=Na 0.95 | 3.03 | 7.85 | 355 | 0.091 |
| *Surface area and pore volumes were determined after a sample was heated under nitrogen at 540°C for 4 hours and then in air at 540°C for 16 hours | | | | | | |

Table 4

| EXAMPLE 2 | | | EXAMPLE 3 | | | EXAMPLE 4 | | |
|---|---|---|---|---|---|---|---|---|
| 2-θ | d(Å) | $I/I_0$% | 2-θ | d(Å) | $I/I_0$% | 2-θ | d(Å) | $I/I_0$% |
| 7.22 | 12.24 | s | 3.04 | 29.00 | w | 3.08 | 28.62 | m |
| 8.58 | 10.30 | s | 6.68 | 13.22 | m | 6.68 | 13.22 | m |
| 20.01 | 4.43 | s | 7.22 | 12.23 | m | 7.21 | 12.25 | m |
| 22.29 | 3.99 | vs | 8.70 | 10.16 | m | 8.76 | 10.08 | m |
| 25.12 | 3.54 | m | 14.63 | 6.05 | w | 14.70 | 6.02 | w |
| 26.04 | 3.42 | vs | 19.98 | 4.44 | vs | 20.11 | 4.41 | m |
| | | | 22.43 | 3.96 | vs | 22.52 | 3.94 | vs |
| | | | 25.03 | 3.55 | w | 25.14 | 3.54 | m |
| | | | 26.02 | 3.42 | s | 26.06 | 3.42 | m-s |
| | | | 26.57 | 3.35 | s | 26.86 | 3.32 | m |
| | | | 31.55 | 2.83 | w | 29.09 | 3.07 | m |
| | | | 33.35 | 2.68 | w | 33.44 | 2.68 | w |
| | | | 37.74 | 2.38 | w | 46.31 | 1.96 | w |
| | | | 46.33 | 1.96 | w | 48.44 | 1.88 | w |
| | | | 48.80 | 1.86 | w | 65.56 | 1.42 | w |
| | | | 51.81 | 1.76 | w | | | |
| | | | 65.46 | 1.42 | w | | | |

Table 5

| EXAMPLE 5 | | | EXAMPLE 6 | | | EXAMPLE 7 | | |
|---|---|---|---|---|---|---|---|---|
| 2-θ | d(Å) | $I/I_0$% | 2-θ | d(Å) | $I/I_0$% | 2-θ | d(Å) | $I/I_0$% |
| 3.00 | 29.39 | s | 3.19 | 27.69 | w | 6.55 | 13.49 | w |
| 6.71 | 13.17 | m | 6.70 | 13.19 | s | 7.13 | 12.38 | m |

(continued)

| EXAMPLE 5 | | | EXAMPLE 6 | | | EXAMPLE 7 | | |
|---|---|---|---|---|---|---|---|---|
| 2-θ | d(Å) | I/I₀% | 2-θ | d(Å) | I/I₀% | 2-θ | d(Å) | I/I₀% |
| 7.16 | 12.34 | m | 7.16 | 12.33 | m | 8.64 | 10.23 | m |
| 8.52 | 10.37 | vs | 8.90 | 9.93 | w | 20.01 | 4.43 | s |
| 13.06 | 6.77 | w | 13.37 | 6.61 | w | 22.47 | 3.95 | vs |
| 14.39 | 6.15 | w | 14.58 | 6.07 | w | 25.04 | 3.55 | w |
| 15.80 | 5.60 | w | 20.06 | 4.42 | vs | 25.94 | 3.43 | m |
| 20.01 | 4.43 | m | 22.41 | 3.96 | m | 26.61 | 3.35 | w |
| 22.18 | 4.00 | vs | 25.06 | 3.55 | w | | | |
| 25.03 | 3.55 | m | 26.04 | 3.42 | s | | | |
| 25.98 | 3.43 | vs | 26.59 | 3.35 | m | | | |
| 26.95 | 3.31 | m | 29.28 | 3.05 | w | | | |
| 28.87 | 3.09 | w-m | 33.58 | 2.67 | w | | | |
| 31.43 | 2.84 | w | 37.90 | 2.37 | w | | | |
| 33.35 | 2.68 | w | 44.77 | 2.02 | w | | | |
| 37.65 | 2.39 | w | 46.25 | 1.96 | w | | | |
| 44.50 | 2.03 | w | 48.60 | 1.87 | w | | | |
| 46.15 | 1.97 | w | 51.90 | 1.76 | w | | | |
| 46.36 | 1.96 | w | 65.34 | 1.43 | w | | | |
| 48.43 | 1.88 | w | | | | | | |
| 51.64 | 1.77 | w | | | | | | |
| 61.04 | 1.52 | w | | | | | | |
| 65.34 | 1.43 | w | | | | | | |

Table 6

| EXAMPLE 8 | | | EXAMPLE 9 | | | EXAMPLE 10 | | |
|---|---|---|---|---|---|---|---|---|
| 2-θ | d(Å) | I/I₀% | 2-θ | d(Å) | I/I₀% | 2-θ | d(Å) | I/I₀% |
| 3.06 | 28.82 | s | 2.86 | 30.91 | vs | 7.26 | 12.17 | m |
| 6.70 | 13.19 | s | 6.63 | 13.32 | w | 8.72 | 10.13 | w |
| 7.21 | 12.26 | s | 7.14 | 12.37 | m | 19.94 | 4.45 | s |
| 8.80 | 10.04 | m | 8.77 | 10.08 | w-m | 22.53 | 3.94 | vs |
| 13.55 | 6.53 | w | 13.54 | 6.54 | w | 25.02 | 3.56 | w |
| 14.74 | 6.00 | w | 14.75 | 6.00 | w | 26.08 | 3.41 | m |
| 20.01 | 4.43 | vs | 16.11 | 5.50 | w | 26.79 | 3.32 | m |
| 22.46 | 3.96 | vs | 19.89 | 4.46 | vs | | | |
| 25.06 | 3.55 | m | 22.42 | 3.96 | s | | | |
| 26.04 | 3.42 | m | 25.00 | 3.56 | m | | | |
| 26.81 | 3.32 | w | 25.96 | 3.43 | s | | | |
| 29.08 | 3.07 | w | 26.60 | 3.35 | m | | | |

(continued)

| EXAMPLE 8 | | | EXAMPLE 9 | | | EXAMPLE 10 | | |
|---|---|---|---|---|---|---|---|---|
| 2-θ | d(Å) | I/I$_0$% | 2-θ | d(Å) | I/I$_0$% | 2-θ | d(Å) | I/I$_0$% |
| 33.53 | 2.67 | w | 33.19 | 2.70 | w | | | |
| 46.27 | 1.96 | w | 37.80 | 2.38 | w | | | |
| 48.66 | 1.87 | w | 46.13 | 1.97 | w | | | |
| 51.83 | 1.76 | w | 51.80 | 1.76 | w | | | |
| 65.34 | 1.43 | w | 65.25 | 1.43 | w | | | |

Table 7

| EXAMPLE 11 | | | EXAMPLE 12 | | | EXAMPLE 13 | | |
|---|---|---|---|---|---|---|---|---|
| 2-θ | d(Å) | I/I$_0$% | 2-θ | d(Å) | I/I$_0$% | 2-θ | d(Å) | I/I$_0$% |
| 6.53 | 13.54 | m | 2.86 | 30.87 | w | 7.04 | 12.55 | w |
| 7.11 | 12.43 | m | 6.40 | 13.80 | m | 8.45 | 10.40 | m |
| 8.58 | 10.30 | vs | 7.08 | 12.48 | m | 19.97 | 4.44 | m |
| 13.06 | 6.77 | w | 8.58 | 10.30 | vs | 22.25 | 3.99 | vs |
| 14.55 | 6.08 | w | 12.73 | 6.95 | w | 24.85 | 3.58 | w |
| 19.88 | 4.46 | s-vs | 14.74 | 6.01 | m | 25.78 | 3.45 | vs |
| 22.35 | 3.97 | s | 19.80 | 4.48 | vs | | | |
| 24.90 | 3.57 | w | 22.20 | 4.00 | vs | | | |
| 25.89 | 3.44 | vs | 24.94 | 3.57 | m | | | |
| 26.96 | 3.30 | m | 25.88 | 3.44 | vs | | | |
| 33.09 | 2.71 | w | 26.86 | 3.32 | m-s | | | |
| 37.75 | 2.38 | w | 33.26 | 2.69 | w | | | |
| 46.26 | 1.96 | w | 36.31 | 2.47 | w | | | |
| 48.68 | 1.87 | w | 37.66 | 2.39 | w | | | |
| 51.81 | 1.76 | w | 46.16 | 1.97 | w | | | |
| 65.26 | 1.43 | w | 48.43 | 1.88 | w | | | |
| | | | 51.90 | 1.76 | w | | | |
| | | | 65.34 | 1.43 | w | | | |

EXAMPLE 14

A 4.78 g portion of a Na gallate solution (9.91 % Ga, 6.31 % Na) was combined with 44.84 g DEDMAOH (20% aq) and to it there were added 19.58 g of colloidal silica and 0.80 g deionized $H_2O$. The reaction mixture was mixed for 20 min and then transferred to a 125mL Teflon-lined autoclave which was placed in an oven set at 150°C and the mixture digested for 10 days at autogenous pressure. The solid product was collected by filtration, washed and dried at 95°C.
Characterization by powder x-ray diffraction identified the product as UZM-8. Characteristic diffraction lines for the product are given in Table 8. By elemental analysis, it was determined that the material consisted of the elemental mole ratios Si/Ga = 12.65, Na/Ga = 0.14, N/Ga = 1.38, and C/N= 5.7. A portion of this material was ammonium ion-exchanged to remove the alkali cations and then heated under $N_2$ at 540°C in $N_2$ for 4 hr followed by heating in air at 540°C for 16 hr. The BET surface area was found to be 333 m$^2$/g and the micropore volume was 0.14 cc/g.

Table 8

| 2-θ | d(Å) | I/I$_0$% |
|---|---|---|
| 3.12 | 28.31 | Vs |
| 6.68 | 13.22 | S |
| 7.12 | 12.41 | Vs |
| 8.63 | 10.24 | M |
| 12.81 | 6.90 | W |
| 15.78 | 5.61 | W |
| 19.96 | 4.45 | S |
| 22.24 | 3.99 | S |
| 24.98 | 3.56 | M |
| 25.92 | 3.43 | Vs |
| 26.60 | 3.35 | M |
| 29.02 | 3.07 | W |
| 31.49 | 2.84 | W |
| 33.37 | 2.68 | M |
| 36.26 | 2.48 | W |
| 37.67 | 2.39 | W |
| 44.67 | 2.03 | W |
| 46.21 | 1.96 | W |
| 48.48 | 1.88 | W |
| 51.90 | 1.76 | W |
| 65.22 | 1.43 | W |

EXAMPLE 15

A first aluminosilicate stock solution was prepared by dissolving 34.76 g of aluminum sec-butoxide in 337.83 g hexamethonium dihydroxide solution (19.75%), followed by the addition of 360.24 g de-ionized water while stirring vigorously. Then 300.0 g of tetraethylorthosilicate (98%) was added and the resulting mixture homogenized for 2 hours. The resulting solution was transferred to a rotary evaporator to remove alcohol from the hydrolysis of the alkoxides.

A second aluminosilicate stock solution was prepared by dissolving 9.93 g of aluminum sec-butoxide in 295.59 g hexamethonium dihydroxide solution, followed by the addition of 413.46 g de-ionized water while stirring vigorously. Then 300.0 g of tetraethylorthosilicate was added and the resulting mixture homogenized for 2 hours. The resulting solution was transferred to a rotary evaporator to remove alcohol from the hydrolysis of the alkoxides.

The synthesis of the zeolite was carried out as follows. A volume of 694 μL of the first aluminosilicate stock solution was pipetted to a teflon reactor. Next, a volume of 356 μL of the second aluminosilicate stock solution was pipetted to the same teflon reactor while mixing on an orbital shaker. Next 20 μL of hexamethonium dihydroxide solution were added, followed by 30 μL of strontium nitrate solution (22.7%). The teflon reactor was then sealed and the reaction mixture homogenized vigorously for an hour and then inserted into an autoclave which was placed into an oven for 120 hours at 175°C. The resulting product was washed, centrifuged, and dried overnight at 75°C.

The x-ray diffraction pattern exhibited the lines characteristic of the material designated UZM-8. Characteristic lines in the diffraction pattern are given in Table 15.

Table 9

| 2-θ | d(Å) | I/I$_0$% |
|---|---|---|
| 6.50 | 13.59 | Vs |

(continued)

| 2-θ | d(Å) | I/I$_0$% |
|---|---|---|
| 7.05 | 12.53 | M |
| 8.67 | 10.19 | M |
| 12.80 | 6.91 | W |
| 14.61 | 6.06 | W |
| 20.00 | 4.44 | S |
| 22.05 | 4.03 | M |
| 24.95 | 3.57 | M |
| 25.95 | 3.43 | S |
| 26.65 | 3.34 | M |
| 33.51 | 2.67 | w |

EXAMPLE 16

This example presents a comparison of MCM-56 versus UZM-8, both as-synthesized and calcined. MCM-56 was prepared according to example 8 of US-A-5,827,491. A sample withdrawn at 60 hrs was washed dried and calcined at 540°C for 4 hr in $N_2$ and 16 hr in air. The UZM-8 samples from examples 1 and 4 were also calcined using the same program. The diffraction lines for each of the calcined samples are given below in Table 10. Figure 1a shows the x-ray diffraction patterns for three as-synthesized samples, while the diffraction patterns for the corresponding calcined patterns are shown in Figure 1b. Both the Figures and Table 10 show that UZM-8 has a different diffraction pattern and thus a different structure from MCM-56.

Table 10

| Calcined MCM-56 and UZM-8 | | | | | | | | |
|---|---|---|---|---|---|---|---|---|
| MCM-56 | | | Example 1 | | | Example 4 | | |
| 2-θ | d(Å) | I/I$_0$% | 2-θ | d(Å) | I/I$_0$% | 2-θ | d(Å) | I/I$_0$% |
| 4.10 | 21.52 | m | 4.38 | 20.17 | m | | | |
| 7.10 | 12.44 | vs | 7.28 | 12.14 | m | 7.34 | 12.03 | m |
| 8.04 | 10.98 | m | 8.76 | 10.08 | s | 8.91 | 9.92 | m |
| 9.76 | 9.06 | vs | | | | | | |
| 12.69 | 6.97 | w | 12.79 | 6.92 | w | 12.84 | 6.89 | w |
| 14.34 | 6.17 | vs | 14.60 | 6.06 | vs | 14.70 | 6.02 | vs |
| 15.89 | 5.57 | m | 16.08 | 5.51 | w | 15.88 | 5.58 | w |
| 19.93 | 4.45 | m | 19.91 | 4.46 | M | 20.19 | 4.40 | w |
| 22.56 | 3.94 | vs | 22.66 | 3.92 | S | 22.82 | 3.89 | vs |
| 23.56 | 3.77 | m | 25.35 | 3.51 | W | | | |
| 25.00 | 3.56 | m | | | | | | |
| 26.04 | 3.42 | vs | 26.31 | 3.38 | M | 26.40 | 3.37 | vs |
| 26.88 | 3.31 | m | 27.04 | 3.31 | M | | | |

EXAMPLE 17

UZM-8, MCM-56, and MCM-22 were compared in decane hydroconversion tests. The UZM-8 used was prepared by the procedure given in Example 5 above, the MCM-56 was prepared according to Example 1 of US-A-5362697, while MCM-22 was prepared according to Example 4 of US-A-4954325, except that the digestion time in the autoclave was shortened to 99 hr at 143°C. The samples were ammonium exchanged, calcined, and impregnated with a suitable amount of tetraamineplatinum dichloride to yield 1 wt.% Pt concentration on the final catalyst. The impregnated zeolites were then screened to 40-60 mesh and then calcined by heating at 250°C for 2 hr. The test was carried out in a high pressure microreactor operating at atmospheric pressure using 500 mg of the meshed samples. The samples were pretreated in a $H_2$ stream for 2 hr at 300°C. The feed consisted of 100:1 $H_2$/decane and the test was conducted over the temperature range from 130°C to 260°C at a 10°C/min ramp rate. Products were analyzed via online GC. The data is summarized in the Table 11 below.

Table 11.

| Comparison of MCM-22, MCM-56, and UZM-8 in Decane Conversion Tests | | | | | | | |
|---|---|---|---|---|---|---|---|
| | Constant Temperature T = 250° C | | | Constant $C_{10}$ Conversion (49%) | | Constant $C_{10}$ Conversion (44%) | |
| Sample | Ex. 5 | MCM-56 | MCM-22 | Ex. 5 | MCM-56 | Ex. 5 | MCM-22 |
| Temp | 250°C | 250°C | 250°C | 218°C | 216°C | 215°C | 208°C |
| $C_{10}$Conv. | 97.7% | 98.9% | 99.8% | 49% | 49% | 44% | 44% |
| MeC$_9$ Isomers | 19.0% | 11.7% | 2.3% | 29.5% | 28.4% | 27.1% | 20.7% |
| EtC$_8$ Isomers | 4.1% | 3.2% | 1.5% | 2.1% | 1.9% | 1.8% | 1.0% |
| Mono-branched $C_{10}$ isomers | 23.1% | 15.0% | 3.8% | 31.6% | 30.3% | 28.9% | 21.7% |
| Di-branched $C_{10}$ isomers | 10.2% | 9.2% | 2.5% | 3.2% | 4.0% | 2.6% | 4.8% |
| $C_{10}$ isomers | 33.3% | 24.2% | 6.3% | 34.7% | 34.3% | 31.5% | 26.5% |
| Cracked products | 64.5% | 74.7% | 93.5% | 14.5% | 15.3% | 13% | 17.8% |

It is easily seen from Table 11 that UZM-8 is not quite as active as MCM-56 or MCM-22, as the decane conversion is lowest for UZM-8 at constant temperature. The order of activity is MCM-22 > MCM-56 > UZM-8. The temperatures required to achieve matching conversion for UZM-8 and the other materials is always higher for UZM-8, again confirming the lower activity. However, it is also seen that UZM-8 favors isomerization vs. MCM-56 and MCM-22. The constant temperature results at 250°C for UZM-8 show significantly more isomerization than either MCM-22 or MCM-56, both of which tend to do a lot more cracking. Direct comparison at constant conversion also shows UZM-8 tends to do more overall isomerization and less cracking than either MCM-56 or MCM-22. Interestingly, while the overall isomerization for UZM-8 is highest among the three materials at constant conversion, UZM-8 formed more mono-branched decane isomers than either MCM-22 or MCM-56, while at the same time MCM-56 and MCM-22 formed more di-branched decane isomers than UZM-8. Hence, UZM-8 has different catalytic properties than both MCM-22 and MCM-56.

EXAMPLE 18

An aluminosilicate reaction mixture was prepared by adding 80.44 g of Al(Osec-Bu)$_3$ to 732.97 g of DEDMAOH with vigorous stirring. This was followed by the addition of 252.7 g of Ultrasil VN SP (85%) silica and then a solution containing 12.67 g NaOH dissolved in 321.22 g distilled water. The mixture was homogenized for 30 minutes, and then 16 g of UZM-8 seeds were added. The reaction mixture was placed in a 2 L stirred autoclave at 150°C for 185 hours. The solid product was isolated by filtration, washed with de-ionized water, and dried at room temperature.

X-ray powder diffraction analysis showed the product to have the UZM-8 structure. Characteristic diffraction lines for the product are shown in Table 13 below. The UZM-8 sample was ammonium ion-exchanged with a solution that contained 1 g $NH_4NO_3$ dissolved in 10 g de-ionized water for every gram of UZM-8. The exchanges was carried out twice, heating for 2 hr at 80°C each time, with thorough washes in between. Characterization of this product is presented in Table 12.

EXAMPLE 19

A solution containing 50 g $HNO_3$ (69%) in 88 g de-ionized water was heated to 98°C before adding to it 23 g of the ammonium exchanged UZM-8 of Example 18. The resulting slurry was stirred for 4 hr at 98°C. The product was isolated by filtration, washed with de-ionized water, and dried at 98°C.

The modified product was determined to be UZM-8HS via x-ray powder diffraction analysis. Characteristic diffraction lines for the product are listed in Table 13. Physical characteristics are presented in Table 12.

Table 12

| Characterization of UZM-8 AND UZM-8HS* | | | |
|---|---|---|---|
| Example # | Si/Al | Surface Area ($m^2$/g) | Pore Volume (cc/g) |
| 18 | 9.47 | 427 | 0.11 |
| 19 | 22.2 | 515 | 0.14 |
| 20 | 20.96 | 504 | 0.14 |
| 21 | 40.58 | 495 | 0.13 |
| 22 | 44.51 | 517 | 0.13 |
| 23 | 24.82 | 449 | 0.12 |
| 24 | 10.51 | 432 | 0.14 |
| 25 | 49.43 | 449 | 0.14 |
| 26 | 80.47 | 452 | 0.15 |
| 27 | 122 | 466 | 0.15 |
| 28 | 11.89 | 514 | 0.13 |
| *Prior to physical measurements, the samples were calcined in $N_2$ for 4 hrs at 540°C. | | | |

Table 13

| EXAMPLE 18 | | | EXAMPLE 19 | | | EXAMPLE 20 | | |
|---|---|---|---|---|---|---|---|---|
| 2-θ | d(Å) | $I/I_o$% | 2-θ | d(Å) | $I/I_o$% | 2-θ | d(Å) | $I/I_o$% |
| 2.88 | 30.61 | m | 4.71 | 18.76 | W | 5.14 | 17.19 | w |
| 6.56 | 13.46 | m | 7.21 | 12.25 | S | 7.10 | 12.44 | s |
| 7.12 | 12.40 | s | 8.58 | 10.30 | Vs | 8.40 | 10.52 | s |
| 8.52 | 10.37 | vs | 14.50 | 6.10 | M | 14.34 | 6.17 | m |
| 12.78 | 6.92 | w | 19.88 | 4.46 | M | 19.62 | 4.52 | m |
| 13.36 | 6.62 | w | 22.50 | 3.95 | Vs | 22.45 | 3.96 | vs |
| 14.39 | 6.15 | w | 25.15 | 3.54 | M | 24.98 | 3.56 | w |
| 19.80 | 4.48 | m | 26.10 | 3.41 | S | 25.96 | 3.43 | s |
| 22.16 | 4.01 | s | 26.82 | 3.32 | M | 33.17 | 2.70 | w |
| 24.90 | 3.57 | m | 33.54 | 2.67 | W | 46.22 | 1.96 | w |
| 25.90 | 3.44 | vs | 46.32 | 1.96 | W | 52.10 | 1.75 | w |
| 26.36 | 3.38 | m | 48.94 | 1.86 | W | 65.25 | 1.43 | w |
| 33.25 | 2.69 | w | 52.12 | 1.75 | W | | | |
| 37.64 | 2.39 | w | 65.73 | 1.42 | W | | | |
| 45.87 | 1.98 | w | | | | | | |
| 48.60 | 1.87 | w | | | | | | |
| 51.53 | 1.77 | w | | | | | | |
| 65.24 | 1.43 | w | | | | | | |

EXAMPLE 20

A solution was prepared by adding 200 g HNO₃ (69%) to 500 g de-ionized water, heating it to 98°C and adding to it 115 g of the ammonium exchanged UZM-8 of Example 18. The resulting slurry was stirred for 18 hr at 98°C. The product was isolated by filtration, washed with de-ionized water, and dried at room temperature.
The product was identified as UZM-8HS via x-ray powder diffraction analysis. Characteristic diffraction lines for the product are listed in Table 13. Physical characteristics are presented in Table 12.

EXAMPLE 21

A solution containing 70 g HNO₃ (69%) and 150 g de-ionized water was heated to 98°C and to it there were added 14 g of the product of Example 20. The slurry was stirred for 7 hr at 98°C. The product was isolated by filtration, washed with de-ionized water, and dried at room temperature.
The product had the UZM-8HS structure as indicated by x-ray powder diffraction analysis. Characteristic lines in the diffraction pattern are given in Table 14. Physical characteristics are presented in Table 12.

EXAMPLE 22

A solution containing 16 g HNO₃ (69%) and 161 g de-ionized water was heated to 98°C and to it there were added 7 g of the double acid extracted UZM-8HS from Example 20. The slurry was stirred for 19 hr at 98°C. The product was isolated by filtration, washed with de-ionized water, and dried at room temperature.
The product was identified as UZM-8HS via powder x-ray diffraction analysis. Characteristic diffraction lines for the product are given in Table 14. Physical characteristics are presented in Table 12.

EXAMPLE 23

A solution containing 50 g HNO₃ (69%) and 88 g de-ionized water was heated to 98°C before adding to it 28 as UZM-8 of (Sn/Al=9.47) that was calcined per Example 8 (See Table 12). The slurry was stirred for 4 hr at 98°C. The product was isolated by filtration, washed with de-ionized water, and dried at 75°C for 12 hr.
The product was identified as UZM-8HS via x-ray powder diffraction anyalysis. Characteristic diffraction lines for the product are listed in Table 14. Physical characteristics are presented in Table 12.

Table 14

| EXAMPLE 21 | | | EXAMPLE 22 | | | EXAMPLE 23 | | |
|---|---|---|---|---|---|---|---|---|
| $2\text{-}\theta$ | d(Å) | $I/I_o$% | $2\text{-}\theta$ | d(Å) | $I/I_o$% | $2\text{-}\theta$ | d(Å) | $I/I_o$% |
| 4.40 | 20.05 | m | 4.52 | 19.56 | vs | 4.30 | 20.56 | vs |
| 6.59 | 13.40 | w | 7.14 | 12.36 | m | 7.14 | 12.37 | m |
| 7.12 | 12.41 | m | 8.46 | 10.44 | m | 8.38 | 10.55 | m |
| 8.46 | 10.45 | s | 12.74 | 6.94 | w | 12.64 | 7.00 | w |
| 12.70 | 6.96 | w | 14.50 | 6.10 | vs | 14.44 | 6.13 | m |
| 14.42 | 6.14 | vs | 19.86 | 4.47 | w | 19.92 | 4.45 | w |
| 19.72 | 4.50 | w | 22.50 | 3.95 | vs | 22.62 | 3.93 | s |
| 22.46 | 3.96 | vs | 25.13 | 3.54 | w | 25.20 | 3.53 | w |
| 25.15 | 3.54 | w | 26.08 | 3.41 | s | 26.07 | 3.42 | m |
| 26.04 | 3.42 | s | 29.30 | 3.05 | w | 33.30 | 2.69 | w |
| 26.92 | 3.31 | m | 32.97 | 2.71 | w | | | |
| 33.36 | 2.68 | w | 52.24 | 1.75 | w | | | |
| 37.96 | 2.37 | w | 65.69 | 1.42 | w | | | |
| 46.63 | 1.95 | w | | | | | | |
| 65.60 | 1.42 | w | | | | | | |

EXAMPLE 24

A UZM-8 composition was prepared as in Example 18 with the physical characteristics set forth in Table 12.

EXAMPLE 25

A solution of 400 g HNO$_3$ (69%) and 67 g H$_2$O was heated to 98°C and to it there were added 113 g of the UZM-8 from Example 24. The resulting slurry was stirred for 11 hr at 98°C. The product was isolated by filtration, washed with de-ionized water, and dried at 98°C.
The product was identified as UZM-8HS via powder x-ray diffraction analysis. Characteristic diffraction lines for the product are listed in Table 15. Physical characteristics are presented in Table 12.

EXAMPLE 26

A solution of 435 g HNO$_3$ (69%) and 14 g de-ionized water was heated to 98°C and to it there was added 70 g of the ammonium exchanged UZM-8. The resulting slurry was stirred for 8.5 hr at 98°C. The product was isolated by filtration, washed with de-ionized water and dried at 98°C.
The product was identified as UZM-8HS via powder x-ray diffraction analysis. Characteristic diffraction lines for the product are listed in Table 15. Physical characteristics are presented in Table 12.

EXAMPLE 27

A solution of 535 g HNO$_3$ (69%) and 14 g de-ionized water was heated to 98°C and to it there were added 70 g of the ammonium exchanged UZM-8. The resulting slurry was stirred for 15 hr at 98°C. The products were isolated by filtration, washed with de-ionized water, and dried at 98°C,
**[0087]** The product was identified as UZM-8HS by powder x-ray diffraction analysis. Characteristic diffraction lines for the product are listed in Table 15.

Table 15

| EXAMPLE 25 | | | EXAMPLE 26 | | | EXAMPLE 27 | | |
|---|---|---|---|---|---|---|---|---|
| 2-θ | d(Å) | I/I$_o$% | 2-θ | d(Å) | I/I$_o$% | 2-θ | d(Å) | I/I$_o$% |
| 4.46 | 19.79 | w | 4.64 | 19.03 | M | 4.34 | 20.34 | w |
| 7.24 | 12.20 | vs | 6.60 | 13.39 | M | 6.53 | 13.53 | w |
| 8.51 | 10.38 | vs | 7.18 | 12.30 | Vs | 7.20 | 12.27 | vs |
| 12.86 | 6.88 | w | 8.47 | 10.43 | Vs | 8.64 | 10.22 | m |
| 14.50 | 6.10 | vs | 12.86 | 6.88 | W | 12.80 | 6.91 | w |
| 19.80 | 4.48 | s | 14.46 | 6.12 | S | 14.48 | 6.11 | w |
| 22.66 | 3.92 | vs | 16.15 | 5.48 | W | 16.10 | 5.50 | w |
| 25.12 | 3.54 | w | 19.89 | 4.46 | W | 19.84 | 4.47 | w |
| 26.18 | 3.40 | vs | 22.67 | 3.92 | Vs | 22.64 | 3.92 | vs |
| 26.86 | 3.32 | m | 25.18 | 3.53 | W | 25.19 | 3.53 | w |
| 29.29 | 3.05 | w | 26.24 | 3.39 | Vs | 26.20 | 3.40 | vs |
| 33.65 | 2.66 | w | 26.88 | 3.31 | M | 26.86 | 3.32 | m |
| 37.90 | 2.37 | w | 33.33 | 2.69 | W | 31.96 | 2.80 | w |
| 46.61 | 1.95 | w | 38.03 | 2.36 | W | 33.72 | 2.66 | w |
| 52.08 | 1.75 | w | 46.36 | 1.96 | W | | | |
| 65.74 | 1.42 | w | 49.23 | 1.85 | W | | | |

EXAMPLE 28

A UZM-8 composition was prepared as in Example 18 and was found to have a Si/Al=10.35 after ammonium exchange. A 3.16 g sample of ammonium hexafluorosilicate was dissolved in 60.10 g de-ionized water. In a separate beaker, 131.45 g of the ammonium exchanged UZM-8 was suspended in 330.5 g de-ionized water and the slurry was heated to 80°C. The ammonium hexafluorosilicate solution was delivered to the zeolite slurry with a pump at a rate of 0.52 cc/min. Once the addition was completed, the slurry was held at 80°C for an additional hour. The product was isolated by filtration and washed with 5 liters of de-ionized water and dried at room temperature.

The product was identified as UZM-8HS via powder x-ray diffraction and elemental analysis. Characteristic diffraction lines for the product are given in Table 16. Physical characteristics are presented in Table 12.

Table 16

| $2\text{-}\theta$ | $d(\text{Å})$ | $I/I_o\%$ |
|---|---|---|
| 6.38 | 13.85 | W |
| 7.08 | 12.48 | S |
| 8.58 | 10.30 | vs |
| 12.68 | 6.98 | W |
| 14.27 | 6.20 | W |
| 15.57 | 5.69 | W |
| 19.76 | 4.49 | M |
| 22.30 | 3.98 | M |
| 24.84 | 3.58 | W |
| 25.90 | 3.44 | S |
| 26.64 | 3.34 | M |
| 31.49 | 2.84 | W |
| 33.02 | 2.71 | W |
| 37.57 | 2.39 | W |

## Claims

1. A microporous crystalline zeolite having a layered framework of at least $AlO_2$ and $SiO_2$ tetrahedral units and a composition on an as-synthesized and anhydrous basis expressed by an empirical formula of:

$$M_m^{n+}R_r^{p+}Al_{1-x}E_xSi_yO_z$$

where M is at least one exchangeable cation selected from the group consisting of alkali and alkaline earth metals, "m" is the mole ratio of M to (Al + E) and varies from 0 to 2.0, R is at least one organoammonium cation selected from the group consisting of quaternary ammonium cations, diquaternary ammonium cations, protonated amines, protonated diamines, protonated alkanolamines and quaternized alkanolammonium cations, "r" is the mole ratio of R to (Al +E) and has a value of 0.05 to 5.0, "n" is the weighted average valence of M and has a value of 1 to 2, "p" is the weighted average valence of R and has a value of 1 to 2, E is an element selected from the group consisting of gallium, iron, boron, chromium, indium and mixtures thereof, "x" is the mole fraction of E and has a value from 0 to 0.50, "y" is the mole ratio of Si to (Al + E) and varies from 6.5 to 35 and "z" is the mole ratio of O to (Al + E) and has a value determined by the equation:

$$z = (m \cdot n + r \cdot p + 3 + 4 \cdot y)/2$$

and is **characterized in that** it has the x-ray diffraction pattern having at least the d spacings and intensities set forth in Table A:

Table A

| 2-θ | d(Å) | I/I$_0$% |
|---|---|---|
| 6.40-6.90 | 13.80-12.80 | w-s |
| 6.95 - 7.42 | 12.70 - 11.90 | m-s |
| 8.33 - 9.11 | 10.60 - 9.70 | w-vs |
| 19.62 - 20.49 | 4.52 - 4.33 | m - vs |
| 21.93 - 22.84 | 4.05 - 3.89 | m - vs |
| 24.71 -25.35 | 3.60 - 3.51 | w - m |
| 25.73 - 26.35 | 3.46 - 3.38 | m - vs |

2. The zeolite of Claim 1 where the zeolite is thermally stable up to a temperature of 600°C.

3. The zeolite of Claim 1 or 2 where R is selected from the group consisting of diethyldimethylammonium, ethyltrimethylammonium, hexamethonium and mixtures thereof.

4. The zeolite of Claim 1 or 2 or 3 where "m" is zero.

5. A microporous crystalline zeolite having a three-dimensional framework of at least SiO$_2$ tetrahedral units and an empirical composition on an anhydrous basis in terms of mole ratios of the elements of:

$$M1_a^{n+} Al_{(1-x)} E_x Si_{y'} O_{z''}$$

where M1 is at least one exchangeable cation selected from the group consisting of alkali metals, alkaline earth metals, rare earth metals, ammonium ion, hydrogen ion and mixtures thereof, a is the mole ratio of M1 to (Al + E) and varies from 0.05 to 5.0, E is an element selected from the group consisting of gallium, iron, boron, chromium, indium and mixtures thereof, x is the mole fraction of E and varies from 0 to 1.0, n is the weighted average valence of M1 and has a value of +1 to +3, y' is the mole ratio of Si to (Al + E) and is greater than 6.5 and z'' is the mole ratio of O to (Al + E) and has a value determined by the equation:

$$z'' = (a \bullet n + 3 + 4 \bullet y')/2$$

the zeolite **characterized in that** it has an x-ray diffraction pattern having at least the d-spacings and relative intensities set forth in Table B:

Table B

| 2-θ | d(Å) | I/I$_o$% |
|---|---|---|
| 6.90 - 7.40 | 12.80 - 11.94 | w-vs |
| 8.15 - 8.85 | 10.84-9.98 | m-vs |
| 14.10 -14.70 | 6.28 - 6.02 | w-vs |
| 19.40 - 20.10 | 4.57 - 4.41 | w-s |
| 22.00 - 22.85 | 4.04 - 3.89 | m-vs |
| 24.65 - 25.40 | 3.61 - 3.50 | w-m |

(continued)

| 2-θ | d(Å) | I/I_o % |
|---|---|---|
| 25.70 - 26.50 | 3.46 - 3.36 | w-vs |

6. A hydrocarbon conversion process comprising contacting a hydrocarbon stream with a catalytic composite at hydrocarbon conversion conditions to give a converted product, the catalytic composite comprising the zeolite of claims 1 or 2 or 3 or 4 or 5.

7. The process of claim 6 where the hydrocarbon conversion process is selected from the group consisting of alkylation of aromatics, isomerization of xylenes, naphtha cracking, ring opening, transalkylation, alkylation of isoparaffins and isomerization of ethyl benzene.

## Patentansprüche

1. Mikroporöser kristalliner Zeolith mit einem schichtförmigen Gerüst aus mindestens $AlO_2$- und $SiO_2$-Tetraedereinheiten und einer Zusammensetzung in der wie bei der Synthese anfallenden Form und auf wasserfreier Basis, ausgedrückt durch die folgende empirische Formel:

$$M_m^{n+}R_r^{p+}Al_{1-x}E_xSi_yO_z$$

worin M für mindestens ein austauschbares Kation aus der Gruppe bestehend aus Alkali- und Erdalkalimetallen steht, "m" für das Molverhältnis von M zu (Al + E) steht und von 0 bis 2,0 variiert, R für mindestens ein Organoammoniumkation aus der Gruppe bestehend aus quartären Ammoniumkationen, diquartären Ammoniumkationen, protonierten Aminen, protonierten Diaminen, protonierten Alkanolaminen und quaternisierten Alkanolammoniumkationen steht, "r" für das Molverhältnis von R zu (Al + E) steht und einen Wert von 0,05 bis 0,5 aufweist, "n" für die gewichtete durchschnittliche Wertigkeit von M steht und einen Wert von 1 bis 2 aufweist, "p" für die gewichtete durchschnittliche Wertigkeit von R steht und einen Wert von 1 bis 2 aufweist, E für ein Element aus der Gruppe bestehend aus Gallium, Eisen, Bor, Chrom, Indium und Mischungen davon steht, "x" für den Molenbruch von E steht und einen Wert von 0 bis 0,50 aufweist, "y" für das Molverhältnis von Si zu (Al + E) steht und von 6,5 bis 35 variiert und "z" für das Molverhältnis von O zu (Al + E) steht und einen durch die folgende Formel bestimmten Wert aufweist:

$$z = (m \cdot n + r \cdot p + 3 + 4 \cdot y)/2$$

**dadurch gekennzeichnet, daß** er das Röntgenbeugungsmuster mit mindestens den folgenden d-Abständen und Intensitäten gemäß Tabelle A aufweist:

Tabelle A

| 2-θ | d (Å) | I/I_0 % |
|---|---|---|
| 6,40-6,90 | 13,80-12,80 | w-s |
| 6,95-7,42 | 12,70-11,90 | m-s |
| 8,33-9,11 | 10,60-9,70 | w-vs |
| 19,62-20,49 | 4,52-4,33 | m-vs |
| 21,93-22,84 | 4,05-3,89 | m-vs |
| 24,71-25,35 | 3,60-3,51 | w-m |
| 25,73-26,35 | 3,46-3,38 | m-vs |

2. Zeolith nach Anspruch 1, wobei der Zeolith bis zu einer Temperatur von 600°C thermisch stabil ist.

**3.** Zeolith nach Anspruch 1 oder 2, wobei R aus der Gruppe bestehend aus Diethyldimethylammonium, Ethyltrimethylammonium, Hexamethonium und Mischungen davon ausgewählt ist.

**4.** Zeolith nach Anspruch 1 oder 2 oder 3, wobei "m" für null steht.

**5.** Mikroporöser kristalliner Zeolith mit einem dreidimensionalen Gerüst aus mindestens $SiO_2$-Tetraedereinheiten und der folgenden empirischen Zusammensetzung auf wasserfreier Basis in Molverhältnissen der Elemente:

$$M1_a^{n+}Al_{(1-x)}E_xSi_{y'}O_{z''}$$

worin M1 für mindestens ein austauschbares Kation aus der Gruppe bestehend aus Alkalimetallen, Erdalkalimetallen, Seltenerdmetallen, Ammoniumion, Wasserstoffion und Mischungen davon steht, a für das Molverhältnis von M1 zu (Al + E) steht und von 0,05 bis 5,0 variiert, E für ein Element aus der Gruppe bestehend aus Gallium, Eisen, Bor, Chrom, Indium und Mischungen davon steht, x für den Molenbruch von E steht und von 0 bis 1,0 variiert, n für die gewichtete durchschnittliche Wertigkeit von M1 steht und einen Wert von +1 bis +3 aufweist, y' für das Molverhältnis von Si zu (Al + E) steht und größer als 6,5 ist und z'' für das Molverhältnis von O zu (Al + E) steht und einen durch die folgende Formel bestimmten Wert aufweist:

$$z'' = (a \cdot n + 3 + 4 \cdot y')/2$$

**dadurch gekennzeichnet, daß** er das Röntgenbeugungsmuster mit mindestens den folgenden d-Abständen und Intensitäten gemäß Tabelle B aufweist:

Tabelle B

| 2-θ | d () | I/I$_0$ % |
|---|---|---|
| 6,90-7,40 | 12,80-11,94 | w-vs |
| 8,15-8,85 | 10,84-9,98 | m-vs |
| 14,10-14,70 | 6,28-6,02 | w-vs |
| 19,40-20,10 | 4,57-4,41 | w-s |
| 22,00-22,85 | 4,04-3,89 | m-vs |
| 24,65-25,40 | 3,61-3,50 | w-m |
| 25,70-26,50 | 3,46-3,36 | w-vs |

**6.** Kohlenwasserstoffumwandlungsverfahren, bei dem man einen Kohlenwasserstoffstrom unter Kohlenwasserstoffumwandlungsbedigungen mit einem katalytisch wirksamen Verbund in Berührung bringt, wobei man ein umgewandeltes Produkt erhält, wobei der katalytisch wirksame Verbund den Zeolith nach den Ansprüchen 1 oder 2 oder 3 oder 4 oder 5 umfaßt.

**7.** Verfahren nach Anspruch 6, bei dem das Kohlenwasserstoffumwandlungsverfahren aus der Gruppe bestehend aus Alkylierung von Aromaten, Isomerisierung von Xylolen, Naphthacracking, Ringöffnung, Umalkylierung, Alkylierung von Isoparaffinen und Isomerisierung von Ethylbenzol ausgewählt wird.

**Revendications**

**1.** Zéolithe cristalline microporeuse possédant une ossature à couches de motifs tétraédriques au moins constitués de $AlO_2$ et $SiO_2$ et une composition, sur une base anhydre au sortir de la synthèse, exprimée par la formule empirique suivante :

$$M_m^{n+}R_r^{p+}Al_{1-x}E_xSi_yO_z$$

dans laquelle M représente au moins un cation échangeable choisi dans le groupe constitué par les métaux alcalins et alcalino-terreux, "m" représente le rapport molaire de M à (Al + E) et varie de 0 à 2,0, R est au moins un cation organoammonium choisi dans le groupe constitué par les cations ammonium quaternaires, les cations diammonium quaternaires, les amines protonées, les diamines protonées, les alcanolamines protonées et les cations alcanolammonium quaternisés, "r" représente le rapport molaire de R à (Al + E) et possède une valeur de 0,05 à 5,0, "n" représente la valence moyenne pondérée de M et possède une valeur de 1 à 2, "p" représente la valence moyenne pondérée de R et possède une valeur de 1 à 2, E représente un élément choisi dans le groupe constitué par le gallium, le fer, le bore, le chrome, l'indium et leurs mélanges, "x" représente la fraction molaire de E et possède une valeur de 0 à 0,50, "y" représente le rapport molaire de Si à (Al + E) et varie de 6,5 à 35 et "z" représente le rapport molaire de O à (Al + E) et possède une valeur déterminée par l'équation :

$$z = (m \bullet n + r \bullet p + 3 + 4 \bullet y) / 2$$

et est **caractérisée en ce qu'**elle présente un spectre de diffraction X possédant au moins les distances réticulaires d et les intensités indiquées dans le tableau A :

Tableau A

| 2 θ | d (Å) | $I/I_0$ % |
|---|---|---|
| 6,40-6,90 | 13,80-12,80 | f - F |
| 6,95-7,42 | 12,70-11,90 | m - F |
| 8,33-9,11 | 10,60-9,70 | f - TF |
| 19,62-20,49 | 4,52-4,33 | m - TF |
| 21,93-22,84 | 4,05-3,89 | m - TF |
| 24,71-25,35 | 3,60-3,51 | f - m |
| 25,73-26,35 | 3,46-3,38 | m - TF |

Tableau A

2. Zéolithe selon la revendication 1 dans laquelle la zéolithe est stable à la chaleur jusqu'à une température de 600°C.

3. Zéolithe selon la revendication 1 ou 2 dans laquelle R est choisi dans le groupe constitué par le diéthyldiméthylammonium, l'éthyltriméthylammonium, l'hexaméthonium et leurs mélanges.

4. Zéolithe selon la revendication 1 ou 2 ou 3 dans laquelle "m" est égal à zéro.

5. Zéolithe cristalline microporeuse possédant une ossature tridimensionnelle de motifs tétraédriques au moins constitués de $SiO_2$ et une composition empirique sur une base anhydre, exprimée en rapports molaires des éléments, de :

$$M1_a^{n+} Al_{(1-x)} E_x Si_{y'} O_{z''}$$

où M1 représente au moins un cation échangeable choisi dans le groupe constitué par les métaux alcalis, les métaux alcalino-terreux, les métaux de terres rares, l'ion ammonium, l'ion hydrogène et leurs mélanges, a représente le rapport molaire de M1 à (Al + E) et varie de 0,05 à 5,0, E représente un élément choisi dans le groupe constitué par le gallium, le fer, le bore, le chrome, l'indium et leurs mélanges, x représente la fraction molaire de E et varie de 0 à 1,0, n représente la valence moyenne pondérée de M1 et possède une valeur de +1 à +3, Y' représente le rapport molaire de Si à (Al + E) et est supérieur à 6,5 et z" représente le rapport molaire de O à (Al + E) et possède une valeur déterminée par l'équation :

$$z" = (a \bullet n + 3 + 4 \bullet y')/2$$

la zéolithe étant **caractérisée en ce qu'**elle présente un spectre de diffraction X possédant au moins les distances réticulaires d et les intensités relatives indiquées dans le tableau B :

Tableau B

| 2 θ | d (Å) | I/I$_0$ % |
|---|---|---|
| 6,90-7,40 | 12,80-11,94 | f-TF |
| 8,15-8,85 | 10,84-9,98 | m-TF |
| 14,10-14,70 | 6,28-6,02 | f-TF |
| 19,40-20,10 | 4,57-4,41 | f-F |
| 22,00-22,85 | 4,04-3,89 | m-TF |
| 24,65-25,40 | 3,61-3,50 | f-m |
| 25,70-26,50 | 3,46-3,36 | f-TF |

6. Procédé de conversion d'hydrocarbures comprenant la mise en contact d'un courant d'hydrocarbures avec un composite catalytique dans des conditions de conversion d'hydrocarbures permettant d'obtenir un produit converti, le composite catalytique comprenant la zéolithe selon les revendications 1 ou 2 ou 3 ou 4 ou 5.

7. Procédé selon la revendication 6 dans lequel le procédé de conversion d'hydrocarbures est choisi dans le groupe constitué par l'alkylation d'aromatiques, l'isomérisation de xylènes, le craquage de naphtas, l'ouverture de cycle, la transalkylation, l'alkylation d'isoparaffines et l'isomérisation d'éthylbenzène.

FIG. 1a

FIG. 1b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5362697 A
- US 5236575 A
- US 4610856 A
- US 5015796 A
- US 4870222 A
- US 5157196 A
- US 5157197 A
- US 5827491 A
- US 4954325 A

### Non-patent literature cited in the description

- *J. Phys. Chem.,* 1996, vol. 100, 3788-3798
- **D. W. Breck.** Zeolite Molecular Sieves. Wiley and Sons, 1974, 441
- **D.W. Breck.** Zeolite Molecular Sieves. John Wiley and Sons, 1974, 636
- **H. Pines.** THE CHEMISTRY OF CATALYTIC HYDROCARBON CONVERSIONS. Academic Press, 1981, 123-154